# EUROPEAN PATENT SPECIFICATION

(11) **EP 1 421 137 B1**
(45) Date of publication and mention of the grant of the patent: **30.04.2008**
(21) Application number: 02734981.0
(22) Date of filing: 21.05.2002
(51) Int. Cl.: C08J 5/18

(54) **LIGHT-DEGRADABLE MATERIAL FOR PRODUCING DISPOSABLE MEDICAL APPLIANCES**
DURCH LICHT ABBAUBARES MATERIAL ZUR HERSTELLUNG VON MEDIZINISCHEN VORRICHTUNGEN ZUM EINMALIGEN GEBRAUCH
MATERIAUX PHOTODEGRADABLES POUR LA PRODUCTION D'APPAREILS MEDICAUX JETABLES

(30) Priority: 09.08.2001 CN 01128331
(43) Date of publication of application: 26.05.2004
(73) Proprietor: Skyland Group Co., Limited, North Point, Hong Kong (CN)
(72) Inventor: JIN, Hua, Beijing 100738 (CN); ZENG, Xiangdong, Wuhan Hubei 430000 (CN); LIU, Jianglin, Beijing 100029 (CN)
(74) Representative: Feakins, Graham Allan
(86) International application number: PCT/CN2002/000343
(87) International publication number: WO 2003/014199

(56) References cited:
- CN-A- 1 088 227
- CN-A- 1 241 588
- US-A- 5 135 966

## Description

### Technical Field

The present invention relates to the material for producing disposable medical appliances, especially relates to the light-degradable material for producing disposable medical appliances.

### Background Art

As the increasing awareness of environment protection, it is limited or forbidden to any production or action which can result in environmental pollution in all over the world. Meanwhile, relevant regulations have been issued. The polypropylene as the light-degradable material is only used for food packing according to the Chinese patent publication No. CN1194275A, which mainly focuses on the international problem of environment pollution i.e. "the white pollution". CN 1 088 227 discloses photodegradable compositions of polyolefins and photosensitizers. However, there is no better solution to deal with the large quantity of usage and discard of disposable medical appliances, such as injectors, dropping bottles, and droppers, those are usually made of polypropylene. While more and more disposable medical appliances are used, the waste medical appliances are left in nature by a large amount. Such medical appliances would decompose in decades, or even longer in nature, because the C-C bonds and C-O bonds in their structure are not sensitive to light. The disposable medical appliances are very useful in preventing from the mutual virus infection. Although it is prescribed in China that all disposable injectors must be sterilized and destroyed on the spot after use, there is no suitable method available to destroy the injectors so that some disposable injectors were used for many times, resulting in severe mutual infection on patients. Therefore it is an important agenda for the governments of each country to prevent the above problem from doing harm to human and pollution to environment. At present, although, the light degradable material is available, it is generally used in food packing. Because the disposable medical appliances require high transparence and high bio-security for the material, the light degradable material for food packing is not suitable for producing disposable medical appliances.

### Disclosure of the Invention

In order to solve above problem that harmfulness brings to people by used disposable medical appliances and pollutant brings to environment, the present invention provides the light-degradable material, obtained from polypropylene resin by adding composite allochromatic agent therein, for producing disposable medical appliances. The allochromatic agent is composed of photoinitiator and sensitizer, wherein the photoinitiator contains ferric citrate (FeC₆H₅O₇) and ferrous pentadione (FeC₅H₈O₂); the sensitizer contains hexachloroacetone (Cl₆C₃O) and acetylbenzene (C₈H₈O). The content of these components is photoinitiator 0.04%~0.08% and sensitizer 0.4%~0.9% based on the weight of polypropylene resin. As in photoinitiator, ferric citrate (FeC₆H₅O₇) is 0.02%~0.04%, ferrous pentadione (FeC₅H₈O₂) is 0.02%~0.04%, and in sensitizer, hexachloroacetone (Cl₆C₃O) is 0.2%~0.45%, acetylbenzene (C₈H₈O) is 0.2%~0.45% based on the weight of the polypropylene resin.

According to the present invention, the disposable medical appliances such as disposable injectors made by light-degradable material remain high transparence, high bio-security, and is insoluble to water. After being used, the medical appliances would change color to yellow while being sterilized by UV rays in short time because of the presence of the composite allochromatic agent.

Reuse of these medical appliances can be thereby avoided. Even when the medical appliances are thrown away in nature, they will keep degrading and decay to powder within 45~75 days while exposing in light. When molecular weight thereof decreases to about 5000, they will decompose to carbon dioxide and water by microorganism in soil as well as the problem of pollution to environment introduced by discarded plastic is resolved. The material is neither toxic nor polluted, and may be as the desired substitute of material for producing disposable medical appliances.

### Examples

### Example 1

Raise the temperature of high speed stirrer to 90°C, and put 125000g of polypropylene, 25g of ferric citrate (FeC₆H₅O₇), 25g of ferrous pentadione (FeC₅H₈O₂), 250g of hexachloroacetone (Cl₆C₃O) and 250g of acetylbenzene (C₈H₈O) into the high speed stirrer, and stirring for 5 minutes at this constant temperature. Then put the mixture into single-screw extruder using automatic feed equipment, and extrude out the pelletings. The heating temperatures of three parts from feed inlet to outlet of single-screw extruder are 140°C, 200°C,125°C respectively.

### Example 2

Raise the temperature of high speed stirrer to 90 °C , put 125000g of polypropylene, 50g of ferric citrate (FeC₆H₅O₇), 50g of ferrous pentadione (FeC₅H₈O₂), 562.5g of hexachloroacetone (Cl₆C₃O) and 562.5g of acetylbenzene (C₈H₈O) into the high speed stirrer, and stirring for 5 minutes at this constant temperature. Then put the mixture into single-screw extruder using automatic feed equipment, and extrude out the pelletings. The heating temperatures of three parts from feed inlet to outlet of single-screw extruder are 140 °C , 200°C ,125°C respectively.

### Example 3

This is the best mode to carry out the invention. Raise the temperature of high speed stirrer to 90°C, put 125000g of polypropylene, 37.5g of ferric citrate (FeC₆H₅O₇), 37.5g of ferrous pentadione (FeC₅H₈O₂), 406.25g of hexafluoroacetone (Cl₆C₃O) and 406.25g of acetylbenzene (C₈H₈O) into the high speed stirrer, and stirring for 5 minutes at this constant temperature. Then put the mixture into single-screw extruder using automatic feed equipment, and extrude out the pelletings. The heating temperatures of three parts from feed inlet to outlet of single-screw extruder are 140°C , 200°C,125°C respectively.

## Claims

1. A light-degradable material for producing disposable medical appliances made of polypropylene resin, **characterised in that** a composite allochromatic agent comprising a photoinitiator and a sensitizer is added to the polypropylene resin, wherein the photoinitiator comprises ferric citrate (FeC₆H₅O₇) and ferrous pentadione (FeC₅H₈O₂) and the sensitizer comprises hexachloroacetone (Cl₆C₃O) and acetylbenzene (C₈H₈O), wherein the content of the photoinitiator is 0.04%~0.08% by weight and the content of the sensitizer is 0.4%~0.9% by weight based on the weight of the polypropylene resin.

2. A material as claimed in claim 1, **characterised in that** the content of the ferric citrate (FeC₆H₅O₇) is 0.02%~0.04% by weight and **in that** the content of the ferrous pentadione (FeC₅H₈O₂) is 0.02%~0.04% by weight based on the weight of the polypropylene resin.

3. A material as claimed in claim 1, **characterised in that** the content of the hexachloroacetone (Cl₆C₃O) is 0.2%~0.45% by weight and **in that** the content of the acetylbenzene (C₈H₈O) is 0.2%~0.45% by weight based on the weight of the polypropylene resin.

## Patentansprüche

1. Durch Licht abbaubares Material zur Herstellung von medizinischen Einweg-Vorrichtungen aus Polypropylen-Harz, **dadurch gekennzeichnet, dass** ein zusammengesetzter allochromatischer Wirkstoff, der einen Fotoinitiator und einen Sensibilisator umfasst, dem Polypropylen-Harz hinzugefügt wird, wobei der Fotoinitiator Eisencitrat (FeC₆H₅O₇) und eisenhaltiges Pentadion (FeC₅H₈O₂) umfasst und der Sensibilisator Hexachloraceton (Cl₆C₃O) und Acetylbenzol (C₈H₈O) umfasst, wobei der Gehalt des Fotoindikators 0,04 - 0,08 Gewichtsprozent und der Gehalt des Sensibilisator 0,4 - 0,9 Gewichtsprozent bezogen auf das Gewicht des Polypropylen-Harzes betragen.

2. Material nach Anspruch 1, **dadurch gekennzeichnet, dass** der Gehalt des Eisencitrats (FeC₆H₅O₇) 0,02 - 0,04 Gewichtsprozent und dass der Gehalt des eisenhaltigen Pentadions (FeC₅H₈O₂) 0,02 - 0,04 Gewichtsprozent bezogen auf das Gewicht des Polypropylen-Harzes betragen.

3. Material nach Anspruch 1, **dadurch gekennzeichnet, dass** der Gehalt des Hexachloracetons(Cl₆C₃O) 0,2 - 0,45 Gewichtsprozent und dass der Gehalt des Acetylbenzols (C₈H₈O) 0,2 - 0,45 Gewichtsprozent bezogen auf das Gewicht des Polypropylen-Harzes betragen.

## Revendications

1. Matériau photodégradable servant à produire des appareils médicaux jetables constitué de résine de polypropylène, **caractérisé en ce qu**'un agent allochromatique composite comprenant un photo-initiateur et un sensibilisateur est ajouté j à la résine de polypropylène, dans lequel le photo-initiateur comporte du citrate ferrique (FeC₆H₅O₇) et du pentadione ferreux (FeC₅H₈O₂) et le sensibilisateur comprend de l'hexachloroacétone (Cl₆C₃O) et de l'acétylbenzène (C₈H₈O), dans lequel la teneur en photo-initiateur est de 0,04% ~ 0,08% en poids et la teneur en sensibilisateur est de 0, 4% ~ 0,9% en poids sur la base du poids de la résine de polypropylène.

2. Matériau selon la revendication 1, **caractérisé en ce que** la teneur en citrate ferrique (FeC₆H₅O₇) est de 0,02% ~ 0,04% en poids et **en ce que** la teneur en pentadione ferreux (FeC₅H₈O₂) est de 0,02% ~ 0,04% en poids sur la base du poids de la résine de polypropylène.

3. Matériau selon la revendication 1, **caractérisé en ce que** la teneur en hexachloroacétone (Cl₆C₃O) est de 0,2% ~ 0,45 % en poids et **en ce que** la teneur de l'acétylbenzène (C₈H₈O) est de 0,2% ~ 0,45% en poids sur la base du poids de la résine de polypropylène.
